# EUROPEAN PATENT SPECIFICATION

(11) **EP 2 818 200 B1**
(45) Date of publication and mention of the grant of the patent: **12.06.2019**
(21) Application number: 14172892.3
(22) Date of filing: 18.06.2014
(51) Int. Cl.: A61N 1/372, A61N 1/05, A61N 1/39

(54) **Conductive intra-body communication for implantable medical devices**
Leitfähige intrakorporale Kommunikation für implantierbare medizinische Vorrichtungen
Communication intra-corps conductrice pour dispositifs médicaux implantables

(30) Priority: 25.06.2013 US 201361838890 P
(43) Date of publication of application: 31.12.2014
(73) Proprietor: BIOTRONIK SE & Co. KG, 12359 Berlin (DE)
(72) Inventor: von Arx, Jeffrey A., Lake Oswego, OR Oregon 97035 (US); Müssig, Dirk, West Linn, OR Oregon 97068 (US); Kraetschmer, Hannes, West Linn, OR Oregon 97068 (US); Homayoun, Habib, Beaverton, OR Oregon 97007 (US); Stotts, Larry, Tigard, OR Oregon 97224 (US)
(74) Representative: Galander, Marcus

(56) References cited:
- WO-A2-2013/082185
- US-B2- 6 704 602

## Description

The invention refers to a medical implantable device adapted for intra-body conductive communications and a method for data communication via electric conductive body tissue.

Conductive communications for implantable medical devices are known inter alia from US 6,704,602 B2 "Implanted Medical Device/External Medical Instrument Communication Utilizing Surface Electrodes" and US 2012/0109236 A1 "Leadless Cardiac Pacemaker with Conducted Communication". In these prior art devices, current pulses are used to represent a logical "1" and a lack of a current pulse is used to represent a logical "0" for conductive data communication.

It is an object of the invention to provide an improved medical device. It is a further object to provide an improved method for communication via electric conductive body tissue.

According to the invention, the first object is achieved by a medical implantable device having a data communication interface and a pulse generator. The data communication interface is operatively connected to the pulse generator. The pulse generator is configured to generate and deliver forward current pulses and reverse current pulses, wherein a polarity of the reverse current pulses is opposite to the polarity of the forward current pulses. The generator is controlled by the data communication interface and the data communication interface together with the pulse generator are configured to generate pulses representing binary digits, wherein a first kind of digits (1 or 0) is represented by a current pulse, and the respective other type of binary digits (0 or 1) is represented by a pause between current pulses. The data communication interface together with the pulse generator are configured to deliver current pulses with strictly alternating polarity so that every other current pulse is a reverse current pulse of opposite polarity compared to an immediately preceding forward current pulse. Thus, every current pulse is both, charge balancing and information encoding.

The medical implantable device is thus applying a bipolar encoding scheme known as alternate mark inversion or modified alternate mark inversion where a binary "0" is encoded as zero volts as in unipolar encoding and a binary "1" is encoded alternately as a positive voltage and a negative voltage

The other object of the invention is achieved by a method for data communication via electric conductive body tissue. In this method, current pulses are used to represent binary digits. The method comprises the steps of:
- Providing time slots, each time slot being provided for one binary digit;
- in a respective time slot, delivering a forward or a reverse current pulse in case the logical "1" is to be transmitted in set time slot or delivering no current pulse in case a logical "0" is to be transmitted in said time slot;
- alternating the polarity of said current pulses so that a next logical "1" is represented by a current pulse of opposite polarity than a respective anteceding current pulse.

By applying this kind of coding scheme in a medical implantable device, a number of benefits are achieved simultaneously: The biggest advantage being that a need for additional charge balancing pulses is avoided, because every other current pulse representing a logical "1" or "0" is balancing the immediately preceding current pulse. Thus, the power requirements for data communication (transmission of data strings) are approximately cut to a half compared to data transmission schemes used in known medical devices, because both, forward current and reverse charge balancing current pulses, are used to communicate information. Since charge balancing has to happen on implantable device electrodes to ensure there is no corrosion, using the charge balancing current to convey information is an innovative way to cut the current draw of conductive communication into roughly half. In a typical implantable device, the charge balancing current happens a fixed time after stimulation. Delaying the charge balancing current until the next logical "1" time slot in conductive communications ensures that charge balancing occurs while utilizing the charge stored in the electrode/tissue interface to power the transmission of that binary digit (bit).

Thus, the device according to the invention and the method according to the invention require less battery capacity to achieve the same result as known implantable devices. Technically, this results in either a smaller-volume implant or in an implant with longer longevity.

In a preferred embodiment of the medical implantable device, the first kind of binary digits is a logical "1" which is represented by a current pulse, and the second kind of binary digits is a logical "0" that is represented by an absence of a current pulse. The current pulses, each representing a logical "1", are of strictly alternating polarity. Thus, the advantages pointed out above are achieved. Alternatively, a logical "0" may be represented by a current pulse, and a logical "1" may be represented by an absence of a current pulse, thus generating a modified alternate mark inversion code.

The data communication interface together with the pulse generator are preferably configured to provide time slots wherein each time slot is provided for representation of one binary digit. The time slots preferably are all of a same duration.

In order to avoid longer periods with no current pulse or with continuous current pulses (either representing a logical "0" or a logical "1", depending on which coding scheme is applied), the medical implantable device, and in particular the data communication interface, preferably is configured to perform a whitening transformation of a data string prior to transmission. Avoiding longer periods of no current pulse, and long periods of continuous current pulses has three advantages: First of all, this ensures that charge balancing occurs in time. Second, clock synchronization of a transmitting and a receiving device is facilitated. Third, the peak current demands from the batter are eased since long consecutive transmit current string are avoided. In a second embodiment a bit balanced encoding schema such as 8B/10B encoding (see US patent 4,486,739) is used rather than data whitening to ensure no long string of consecutive "1"s or "0"s.

The pulse generator preferably comprises pulse delivery circuitry, comprising a capacitor through which a forward current pulse is delivered so that the capacitor is charged upon delivery of a forward current pulse. The pulse delivery circuitry further comprises at least one switch that allows discharging of the capacitor in order to generate a reverse current pulse. The capacitor thus effectively causes charge balancing and data transmission uno acto.

According to a further preferred embodiment, the pulse generator is configured to deliver forward current pulses and backward current pulses, which each comprise an at least nearly same amount of charge to ensure charge neutrality on the electrodes. The electrodes preferably are surface electrodes of the implantable medical device or electrodes connected to the implantable medical device.

The medical implantable device further is preferably configured to generate and deliver sub-threshold current pulses for data communications. The current pulses can be sub-threshold because they are low amplitude, narrow pulse width, high frequency, or some combination of these. Thus, unintended tissue stimulation is avoided.

Preferably, the medical implantable device is a stimulation device that is capable of delivering both, sub-threshold current pulses for data communications and supra-threshold current pulses for stimulation. In particular, the medical implantable device may be a cardiac pacemaker, an implantable cardioverter/defibrillator (ICD) or the like.

According to preferred embodiments of the method, prior to transmission of data strings, the whitening transformation of the data strings to be transmitted is performed.

The time slots provided in the method preferably each have a same duration to facilitate clock synchronization.

The current pulses delivered according to the method preferably each comprise an essentially same amount of charge in order to achieve charge neutrality over time.

According to a particularly preferred embodiment of the method, each forward current pulse is delivered via a capacitor, so that the capacitor is charged upon delivery of a respective forward current pulse, and a reverse current pulse is effected by discharging the capacitor.

The forward current pulses and reverse current pulses preferably are sub-threshold current pulses having an intensity below a capture threshold of body tissue in order to avoid unintended tissue stimulation.

Preferably, every other current pulse is a reverse current pulse to thus achieve a sequence of current pulses of strictly alternating polarity.

The above and other aspects, features and advantages of the present invention will be more apparent from the following more particular description thereof, presented in conjunction with the following drawings wherein:
- Fig. 1: is a schematic representation of an implantable leadless pacemaker (iLP).
- Fig. 2: is a schematic explodes view of the pacemaker in Figure 1.
- Fig. 3: is schematic block diagram of a data communications interface and a communication pulse generator.
- Fig. 4: illustrates details of the pulse generator and its pulse delivery circuitry.
- Fig. 5: illustrates intra-body communication between two implantable leadless pacemakers that together act as two chamber pacemaker.

The following description is of the best mode presently contemplated for carrying out the invention. This description is not to be taken in a limiting sense, but is made merely for the purpose of describing the general principles of the invention. The scope of the invention should be determined with reference to the claims.

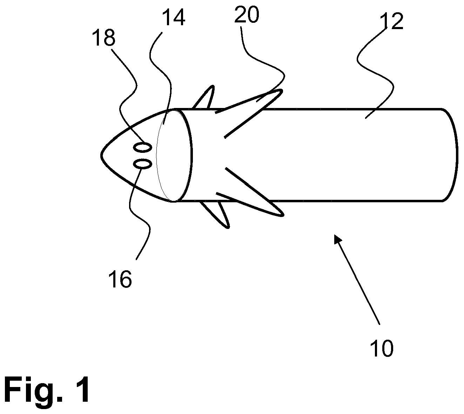

Fig. 1 illustrates an implantable leadless pacemaker 10 that is an implantable medical (stimulation) device. Pacemaker 10 exhibits an elongated cylindrical case 12 with a hemispherical cap 14. Close to the apex of cap 14, two small surface sensing and stimulation electrodes 16 and 18 are arranged. Alternatively electrode 16 is located at the distal end of the pacemaker and electrode 18 is located at the proximal end. Hooks or tines 20 allow an anchoring of pacemaker 10 in a heart chamber.

Case 12 is tightly attached to cap 14 in order form a hermetically closed housing. Electrodes 16 and 18 are integrated in cap 14 and exhibit an electrically conducting surface that is electrically isolated with respect to the housing. In alternative embodiments case 12 is formed as hermetically closed housing with isolated electrodes 16 and 18 at the distal and proximal end of the case respectively. Electrodes 16 and 18 are surface electrodes that serve as poles for stimulation of heart tissue. Electrodes 16 and 18 can also be used as electrode for conductive intra-body communications. The housing of pacemaker 10, and in particular its can 12, can also act as a large surface electrode for conductive intra-body communication.

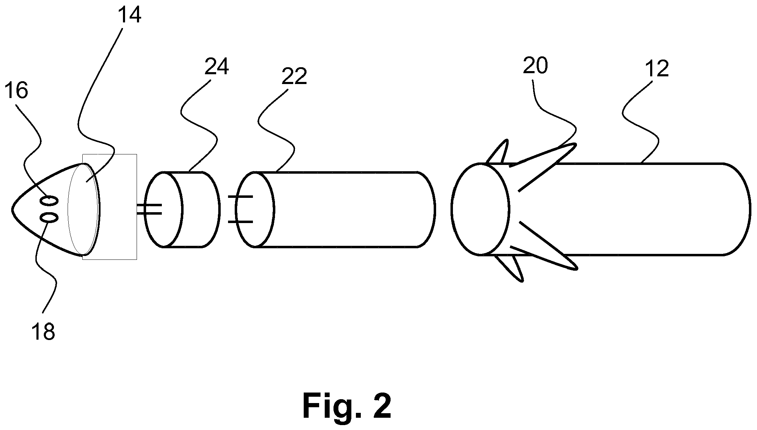

In Fig. 2, an exploded view of pacemaker 10 is illustrated to show components that are enclosed by pacemaker can 12. These components comprise a battery 22 and an electronics module 24. The electronics module 24 comprises pacemaker control electronics and a stimulation pulse generator and, in case of a demand pacemaker, further sensing units for picking up and processing electric potentials from heart tissue are provided.

The implantable leadless pacemaker 10 has a very small battery 22 (∼0.5 cm³) with a minute capacity (∼200 mAh). In traditional pacemakers telemetry is one of the major consumers of the battery capacity. In the implantable leadless pacemaker 10 a conductive communication technique according to the invention is used for telemetry to help minimize the impact on the battery.

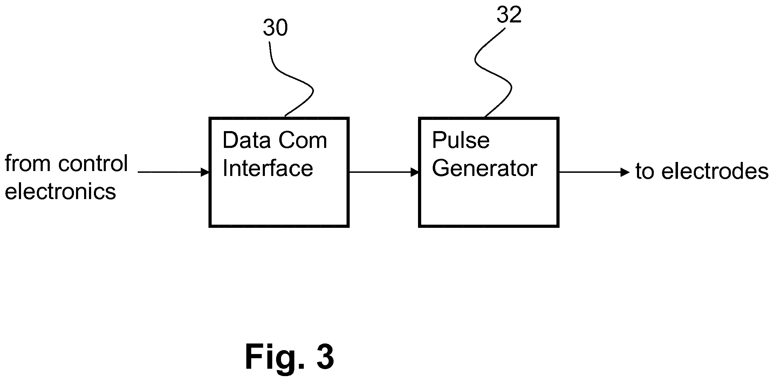

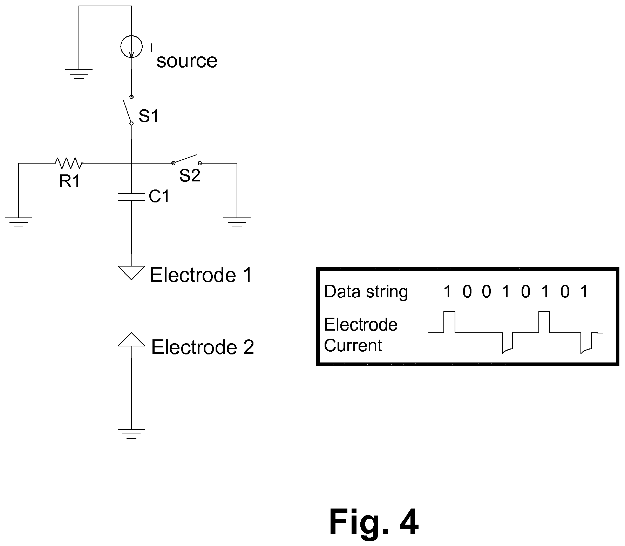

Electronics module 24 further comprises a data communication interface 30 and a communication pulse generator 32 comprising pulse delivery circuitry as shown in Figs. 3 and 4.

As can be taken from Figure 4, pulse delivery circuitry comprises a current source I_{source} connected to a capacitor C1 via first switch S1. An exemplary example for the current source is a constant current source as known in the art. In an alternative embodiment the current source I_{source} is replaced by a voltage source connected to a capacitor C1 via first switch S1. The voltage source provides a voltage and is designed to have an internal resistance that limits the maximum current drawn. Thus, pulse delivery circuitry is configured to generate and deliver electrical pulses, that are either current pulses or voltage pulses. Capacitor C1 is also connected to a first medical device surface electrode 1 such as electrode 16 or 18. A second switch S2 allows fast discharging the capacitor C1 via electrode 1. An optional resistor R1 has relatively high ohmic resistance (e.g. 200 kΩ) and allows slow discharging of capacitor C1 in order to avoid unintended charge build-up.

In case of data communication when the implant (10) is transmitting, a data string comprised of binary digits (bits; logical "1" and "0") is put out by the pacemaker control electronics and received by the data communications interface.

Data communications interface 30 performs a data whitening on received data string in order to avoid long strings of logical "0"s in the data to be transmitted.

Every time an implanted medical device outputs current or voltage through electrodes, a charge balancing current is needed to ensure that the total charge through the electrode is neutral. This is required to minimize electrode corrosion, and it is required by standards such as EN 45502-2-1. In the implantable medical device according to the invention, the power required to transmit can be minimized by utilizing both the forward current or voltage and the charge balancing current or voltage as communication bits. This technique reduces the power required to transmit data by roughly half.

To use this technique, an implantable medical device according to the invention sends a current or voltage pulse of alternating polarity for a logical "1" and no current or voltage for a logical "0". The 1^{st} logical "1" bit is sent as a forward current or voltage pulse. This forward current or voltage pulse goes through a charge balancing capacitor on its way to the electrode. The forward current or voltage induces a charge on the charge balancing capacitor. The pulse generator or pulse delivery circuitry, respectively, of the implantable medical device then holds this charge on the capacitor until the transmission time slot for the next logical "1" bit. During this time slot, the pulse generator or pulse delivery circuitry discharges the charge balancing capacitor through the electrodes. This induces a current or voltage through the electrodes of the opposite polarity of the initial forward current or voltage, and this reverse current or voltage comes at no additional energy cost (except for the relatively small current needed to drive the transmission logic). Thus, in the device and in the method, both, forward current or voltage pulses and charge balancing (reverse) current or voltage pulses are used to convey data. This reduces by approximately ½ the transmit power requirements of the implantable medical device.

To insure that charge balancing is maintained through the electrodes, the implantable medical device has a moderately high impedance leakage path via resistor R1 to allow for any residual change on the charge balancing capacitor to bleed off though the electrodes. This will ensure charge balancing is maintained even if the communication windows end up with more charge flowing in one direction than the other.

A data whitening algorithm is preferably used to ensure that there are not long runs of logical "0"s which might otherwise allow too much charge to bleed off the charge balancing capacitor in-between logical "1" bits.

This invention will roughly cut in half the current consumed intra-body conductive telemetry, and this in turn will reduce significantly the size of the battery needed, which in turn will reduce significantly the size of the implantable leadless pacemaker device.

With respect to Figure 4 operation of the pulse generator and its pulse delivery circuitry is illustrated. Data Whitening is used to ensure that there are no long strings of logical "1"s or "0"s in the data to be transmitted. When the first logical "1" is to be transmitted, switch S1 is closed, and the current I_{source} flows through capacitor C1, and then through electrodes 1 and 2. At the end of the time slot for the first bit, S1 is opened and current stops flowing through the electrodes 1 and 2 At this point capacitor C1 has been charged up by the current and is holding the charge. The next time a logical "1" is to be transmitted, switch S2 is closed at the start of the bit window. Now the capacitor C1 discharges through the electrodes 1 and 2 resulting in current though the body of opposite polarity from the 1^{st} logical "1" bit. At the end of the time slot for this bit, switch S2 is opened and the current stops flowing through the electrodes. One embodiment includes the resistor R1, which is a large value (∼200 kΩ) and is there to ensure that over time no residual charge builds up on the capacitor, and that should the data packet contain an odd number of logical "1"s, the charge from the last logical "1" has a path to dissipate through the electrodes. In another embodiment switch S2 is closed for an extended period of time (more than one bit window) at the end of each transmitted data packet. This ensures that any residual charge left of the capacitor C1 has time to discharge.

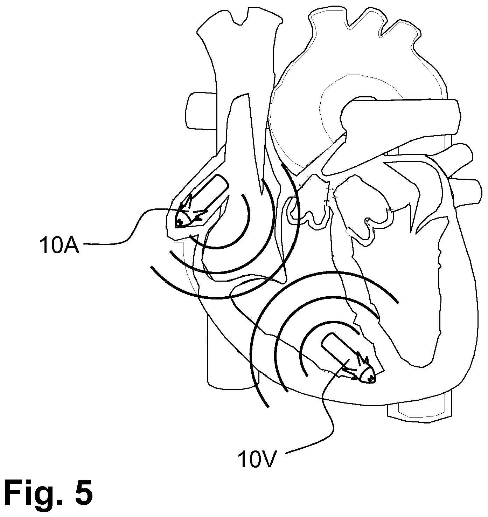

Using the technique in this invention will half the required battery current (to a first order) because the negative current pulse uses residual charge stored on the charge balancing capacitance rather than charge from the battery for power. Because the invention roughly cuts in half the battery capacity needed to support conductive intra-body communications very small implantable systems such as intracardiac leadless pacermaker are made possible. This further enables multi-chamber pacing systems comprising a plurality of implantable leadless pacemakers 10A (in the right atrium of a heart) and 10V (in the right ventricle of a heart) as illustrated in Figure 5.

## Claims

1. Medical implantable device (10) having a data communication interface (30) and a pulse generator (32), wherein the data communication interface (30) is operatively connected to the pulse generator (32), said pulse generator (32) being configured to generate and deliver forward electrical pulses and reverse electrical pulses wherein a polarity of the reverse electrical pulses is opposite to the polarity of the forward electrical pulses, said pulsed generator being controlled by said data communication interface (30), said data communication interface (30) together with said pulse generator (32) being configured to generate said electrical pulses such that they represent binary digits, **characterised in that** a first kind of digits (1 or 0) is represented by said electrical pulse and the respective other type of digits (0 or 1) is represented by a pause between said electrical pulses, wherein said data communication interface (30) together with said pulse generator (32) is further configured to deliver said electrical pulses with strictly alternating polarity so that every other electrical pulse is a reverse electrical pulse of opposite polarity compared to an immediately preceding forward electrical pulse.

2. Medical implantable device according to claim 1, wherein the first kind of binary digits represents a logical "1" and is represented by a electrical pulse and the second kind of binary digits represents a logical "0" and is represented by an absence of a electrical pulse.

3. Medical implantable device according to claim 1 or 2, wherein said data communication interface (30) together with said pulse generator (32) being configured to provide timeslots, each timeslot being provided for a representation of one binary digit.

4. Medical implantable device according to at least one of claims 1 to 3, wherein said data communication interface (30) is configured to perform a whitening transformation on a data string prior to transmission.

5. Medical implantable device according to at least one of claims 1 to 4, wherein aid pulse generator (32) comprises pulse delivery circuitry comprises a capacitor (C1) through which a forward electrical pulse is delivered so that the capacitor (C1) is charged upon delivery of a forward electrical pulse, said capacitor (C1) being discharged when a reverse electrical pulse is delivered.

6. Medical implantable device according to at least one of claims 1 to 5, wherein said pulse generator (32) is configured to deliver forward electrical pulse and backward electrical pulses which each comprise an at least nearly same amount of charge.

7. Medical implantable device according to at least one of claims 1 to 6, said medical implantable device (10) being configured to generate and deliver sub-threshold electrical pulses for data communication and supra threshold electrical pulses for stimulation.

8. Method for data communication via electric conductive body tissue, said method comprising delivery of electrical pulses representing binary digits, said method further comprising
- providing time slots, each time slot being provided for one binary digit, **characterised by** further comprising
- in a respective time slot, delivering a forward or a reverse electrical pulse in case a logical "1" is to be transmitted in said time slot or delivering no electrical pulse in case a logical "0" is to be transmitted in said time slot
- alternating the polarity of said electrical pulses so that a next logical "1" is represented by a current or voltage of opposite polarity than a respective anteceding electrical pulse.

9. Method for data communication via electric conductive body tissue, said method comprising delivery of electrical pulses representing binary digits, said method further comprising
- providing time slots, each time slot being provided for one binary digit
- in a respective time slot, delivering a forward or a reverse electrical pulse in case a logical "0" is to be transmitted in said time slot or delivering no electrical pulse in case a logical "1" is to be transmitted in said time slot
- alternating the polarity of said electrical pulses so that a next logical "0" is represented by a current or voltage of opposite polarity than a respective anteceding electrical pulse.

10. Method for data communication via electric conductive body tissue according to claim 8 or 9, wherein a data string comprising binary digits to be transmitted is whitening transformed prior to transmission.

11. Method for data communication via electric conductive body tissue according to claim 8 or 9, wherein said time slots each have a same duration.

12. Method for data communication via electric conductive body tissue according to claim 8 or 9, wherein said electrical pulses comprise an essentially same amount of charge.

13. Method for data communication via electric conductive body tissue according to claim 8 or 9, wherein a forward electrical pulse is delivered via capacitor through so that the capacitor is charged upon delivery of a forward electrical pulse, and wherein a reverse electrical pulse is effected by discharging said capacitor.

14. Method for data communication via electric conductive body tissue according to claim 8 or 9, wherein said electrical pulses representing binary digits for data communication are sub-threshold electrical pulses having an intensity below a capture threshold of body tissue.

15. Method for data communication via electric conductive body tissue according to claim 8 or 9, wherein the electrical pulses are either current pulses or voltage pulses.

## Patentansprüche

1. Medizinisches implantierbares Gerät (10) mit einer Datenübertragungsschnittstelle (30) und einem Pulsgenerator (32), wobei die Datenübertragungsschnittstelle (30) funktionell mit dem Pulsgenerator (32) verbunden ist, wobei der Pulsgenerator (32) zum Erzeugen und Abgeben von elektrischen Vorwärtspulsen und elektrischen Rückwärtspulsen konfiguriert ist, wobei eine Polarität der elektrischen Rückwärtspulse der Polarität der elektrischen Vorwärtspulse entgegengesetzt ist, wobei der gepulste Generator durch die Datenübertragungsschnittstelle (30) gesteuert wird, wobei die Datenübertragungsschnittstelle (30) zusammen mit dem Pulsgenerator (32) zum Erzeugen der elektrischen Pulse, so dass sie Binärziffern darstellen, konfiguriert ist, **dadurch gekennzeichnet, dass**
eine erste Art von Ziffern (1 oder 0) durch den elektrischen Puls dargestellt wird und der jeweilige andere Zifferntyp (0 oder 1) durch eine Pause zwischen den elektrischen Pulsen dargestellt wird, wobei die Datenübertragungsschnittstelle (30) zusammen mit dem Pulsgenerator (32) ferner zum Abgeben der elektrischen Pulse mit wechselnder Polarität konfiguriert ist, so dass jeder zweite elektrische Puls verglichen mit einem unmittelbar vorangehenden elektrischen Vorwärtspuls ein elektrischer Rückwärtspuls entgegengesetzter Polarität ist.

2. Medizinisches implantierbares Gerät nach Anspruch 1, wobei die erste Art von Binärziffern eine logische "1" darstellt und durch einen elektrischen Puls dargestellt wird und die zweite Art von Binärziffern eine logische "0" darstellt und durch Fehlen eines elektrischen Pulses dargestellt wird.

3. Medizinisches implantierbares Gerät nach Anspruch 1 oder 2, wobei die Datenübertragungsschnittstelle (30) zusammen mit dem Pulsgenerator (32) zum Bereitstellen von Zeitfenstern konfiguriert ist, wobei jedes Zeitfenster für eine Darstellung einer Binärziffer bereitgestellt wird.

4. Medizinisches implantierbares Gerät nach wenigstens einem der Ansprüche 1 bis 3, wobei die Datenübertragungsschnittstelle (30) zum Durchführen einer Whitening-Transformation an einer Datenreihe vor der Übertragung konfiguriert ist.

5. Medizinisches implantierbares Gerät nach wenigstens einem der Ansprüche 1 bis 4, wobei der Pulsgenerator (32) eine Pulsabgabeschaltungsanordnung aufweist, die einen Kondensator (C1) aufweist, durch den ein elektrischer Vorwärtspuls abgegeben wird, so dass der Kondensator (C1) bei Abgabe eines elektrischen Vorwärtspulses aufgeladen wird, wobei der Kondensator (C1) entladen wird, wenn ein elektrischer Rückwärtspuls abgegeben wird.

6. Medizinisches implantierbares Gerät nach wenigstens einem der Ansprüche 1 bis 5, wobei der Pulsgenerator (32) zur Abgabe eines elektrischen Vorwärtspulses und elektrischer Rückwärtspulse konfiguriert ist, die jeweils eine zumindest fast gleiche Ladungsmenge aufweisen.

7. Medizinisches implantierbares Gerät nach wenigstens einem der Ansprüche 1 bis 6, wobei das medizinische implantierbare Gerät (10) zum Erzeugen und Abgeben von unterschwelligen elektrischen Pulsen zur Datenübertragung und überschwelligen elektrischen Pulsen zur Stimulation konfiguriert ist.

8. Verfahren zur Datenübertragung über elektrisch leitendes Körpergewebe, wobei das Verfahren die Abgabe von elektrischen Pulsen, die Binärziffern darstellen, aufweist, wobei das Verfahren ferner umfasst
- Bereitstellen von Zeitfenstern, wobei jedes Zeitfenster für eine Binärziffer bereitgestellt wird,
**dadurch gekennzeichnet, dass** es ferner aufweist
- in einem jeweiligen Zeitfenster Abgeben eines elektrischen Vorwärts- oder Rückwärtspulses im Fall, dass in dem Zeitfenster eine logische "1" zu übertragen ist, oder Abgeben keines elektrischen Pulses im Fall, dass in dem Zeitfenster eine logische "0" zu übertragen ist,
- Wechseln der Polarität der elektrischen Pulse, so dass eine nächste logische "1" durch einen Strom oder eine Spannung mit einer Polarität, die einem jeweiligen vorangehenden elektrischen Puls entgegengesetzt ist, dargestellt wird.

9. Verfahren zur Datenübertragung über elektrisch leitendes Körpergewebe, wobei das Verfahren das Abgeben von Binärziffern darstellenden elektrischen Pulse, wobei das Verfahren ferner aufweist
- Bereitstellen von Zeitfenstern, wobei jedes Zeitfenster für eine Binärziffer bereitgestellt wird,
- in einem jeweiligen Zeitfenster Abgeben eines elektrischen Vorwärts- oder Rückwärtspulses im Fall, dass in dem Zeitfenster eine logische "0" zu übertragen ist, oder Abgeben keines elektrischen Pulses im Fall, dass in dem Zeitfenster eine logische "1" zu übertragen ist,
- Wechseln der Polarität der elektrischen Pulse, so dass eine nächste logische "0" durch einen Strom oder eine Spannung mit einer Polarität, die einem jeweiligen vorangehenden elektrischen Puls entgegengesetzt ist, dargestellt wird.

10. Verfahren zur Datenübertragung über elektrisch leitendes Körpergewebe nach Anspruch 8 oder 9, wobei eine zu übertragende Binärziffern umfassende Datenreihe vor der Übertragung durch eine Whitening-Transformation transformiert wird.

11. Verfahren zur Datenübertragung über elektrisch leitendes Körpergewebe nach Anspruch 8 oder 9, wobei die Zeitfenster jeweils die gleiche Dauer haben.

12. Verfahren zur Datenübertragung über elektrisch leitendes Körpergewebe nach Anspruch 8 oder 9, wobei die elektrischen Pulse eine im Wesentlichen gleiche Ladungsmenge aufweisen.

13. Verfahren zur Datenübertragung über elektrisch leitendes Körpergewebe nach Anspruch 8 oder 9, wobei ein elektrischer Vorwärtspuls durch einen Kondensator hindurch abgegeben wird, so dass der Kondensator bei Abgabe eines elektrischen Vorwärtspulses aufgeladen wird, und wobei ein elektrischer Rückwärtspuls durch Entladung des Kondensators bewirkt wird.

14. Verfahren zur Datenübertragung über elektrisch leitendes Körpergewebe nach Anspruch 8 oder 9, wobei die elektrischen Pulse, die Binärziffern zur Datenübertragung darstellen, unterschwellige elektrische Pulse sind, die eine Stärke unter einer Reizschwelle von Körpergewebe haben.

15. Verfahren zur Datenübertragung über elektrisch leitendes Körpergewebe nach Anspruch 8 oder 9, wobei die elektrischen Pulse entweder Strompulse oder Spannungspulse sind.

## Revendications

1. Dispositif médical implantable (10) ayant une interface de communication de données (30) et un générateur d'impulsions (32), l'interface de communication de données (30) étant reliée de manière opérationnelle au générateur d'impulsions (32), ledit générateur d'impulsions (32) étant configuré pour générer et délivrer des impulsions électriques directes et des impulsions électriques inverses, où une polarité des impulsions électriques inverses est opposée à la polarité des impulsions électriques directes, ledit générateur d'impulsions étant commandé par ladite interface de communication de données (30), ladite interface de communication de données (30) étant configurée conjointement avec ledit générateur d'impulsions (32) pour générer lesdites impulsions électriques de sorte qu'elles représentent des chiffres binaires,
**caractérisé en ce**
**qu'**une première espèce de chiffre (1 ou 0) est représentée par ladite impulsion électrique et l'autre type respectif de chiffre (0 ou 1) est représenté par une pause entre lesdites impulsions électriques, où ladite interface de communication de données (30), conjointement avec ledit générateur d'impulsions (32), est en outre configurée pour délivrer lesdites impulsions électriques avec une polarité strictement alternative, de sorte que chaque nouvelle impulsion électrique est une impulsion électrique inverse, de polarité opposée comparativement à une impulsion électrique directe immédiatement précédente.

2. Dispositif médical implantable selon la revendication 1, dans lequel la première espèce de chiffres binaires représente un « 1 » logique et est représentée par une impulsion électrique et la seconde espèce de chiffres binaires représente un « 0 » logique et est représentée par une absence d'impulsion électrique.

3. Dispositif médical implantable selon la revendication 1 ou la revendication 2, dans lequel ladite interface de communication de données (30), conjointement avec le générateur d'impulsions (32), étant configurés pour produire des créneaux horaires, chaque créneau horaire étant prévu pour une représentation d'un chiffre binaire.

4. Dispositif médical implantable selon au moins une quelconque des revendications 1 à 3, dans lequel ladite interface de communication de données (30) est configurée pour effectuer une transformation de blanchiment sur une chaîne de données avant la transmission.

5. Dispositif médical implantable selon au moins une quelconque des revendications 1 à 4, dans lequel ledit générateur d'impulsions (32) comprend un circuit de délivrance d'impulsions, comprend un condensateur (C1) par lequel une impulsion électrique directe est délivrée, de sorte que le condensateur (C1) est chargé lors de la délivrance d'une impulsion électrique directe, ledit condensateur (C1 étant déchargé lorsqu'une impulsion électrique inverse est délivrée.

6. Dispositif médical implantable selon au moins une quelconque des revendications 1 à 5, dans lequel ledit générateur d'impulsions (32) est configuré pour délivrer une impulsion électrique directe et des impulsions électriques indirectes qui comprennent chacune au moins une quantité de charge pratiquement pareille.

7. Dispositif médical implantable selon au moins une quelconque des revendications 1 à 6, dans lequel ledit dispositif médical implantable (10) étant configuré pour générer et délivrer des impulsions électriques infraliminaires pour la communication de données et des impulsions électriques supraliminaires pour la stimulation.

8. Procédé de communication de données par le biais de tissu corporel conducteur électrique, ledit procédé comprenant la délivrance d'impulsions électriques représentant des chiffres binaires, ledit procédé comprenant en outre
- la mise en place de créneaux horaires, chaque créneau horaire étant produit pour un chiffre binaire,
caractérisé en comprenant en outre
dans un créneau horaire respectif, la délivrance d'une impulsion électrique directe ou inverse dans le cas où un « 1 » logique doit être transmis dans ledit créneau horaire, ou la délivrance d'aucune impulsion électrique dans le cas où un « 0 » logique doit être transmis dans ledit créneau horaire,
- l'alternance de la polarité desdites impulsions électriques de sorte qu'un « 1 » logique prochain est représenté par un courant ou une tension de polarité opposée à l'impulsion électrique précédente respective.

9. Procédé de communication de données par le biais de tissu corporel conducteur électrique, ledit procédé comprenant la délivrance d'impulsions électriques représentant des chiffres binaires, ledit procédé comprenant en outre
- la mise en place de créneaux horaires, chaque créneau horaire étant produit pour un chiffre binaire,
- dans un créneau horaire respectif, la délivrance d'une impulsion électrique directe ou inverse dans le cas où un « 1 » logique doit être transmis dans ledit créneau horaire, ou la délivrance d'aucune impulsion électrique dans le cas où un « 0 » logique doit être transmis dans ledit créneau horaire,
- l'alternance de la polarité desdites impulsions électriques de sorte qu'un « 1 » logique prochain est représenté par un courant ou une tension de polarité opposée à l'impulsion électrique précédente respective.

10. Procédé de communication de données par le biais de tissu corporel conducteur électrique selon la revendication 8 ou la revendication 9, dans lequel une chaîne de données comprenant des chiffres binaires devant être transmis est transformée par blanchiment avant la transmission.

11. Procédé de communication de données par le biais de tissu corporel conducteur électrique selon la revendication 8 ou la revendication 9, dans lequel lesdits créneaux horaires ont chacun la même durée.

12. Procédé de communication de données par le biais de tissu corporel conducteur électrique selon la revendication 8 ou la revendication 9, dans lequel lesdites impulsions électriques comprennent une quantité essentiellement identique de charge.

13. Procédé de communication de données par le biais de tissu corporel conducteur électrique selon la revendication 8 ou la revendication 9, dans lequel une impulsion électrique est délivrée par un condensateur par le fait que le condensateur est chargé lors de la délivrance d'une impulsion électrique directe et dans lequel une impulsion électrique inverse est effectuée en déchargeant ledit condensateur.

14. Procédé de communication de données par le biais de tissu corporel conducteur électrique selon la revendication 8 ou la revendication 9, dans lequel lesdites impulsions électriques représentant des chiffres binaires pour la communication de données sont des impulsions électriques infraliminaires ayant une intensité inférieure à un seuil de détection du tissu corporel.

15. Procédé de communication de données par le biais de tissu corporel conducteur électrique selon la revendication 8 ou la revendication 9, dans lequel les impulsions électriques sont soit des impulsions de courant, soit des impulsions de tension.
